(19) Europäisches Patentamt
European Patent Office
Office européen des brevets

(11)  EP 4 240 174 B1

(12)  EUROPEAN PATENT SPECIFICATION

(45) Date of publication and mention
of the grant of the patent:
**24.07.2024 Bulletin 2024/30**

(51) International Patent Classification (IPC):
***A23K 20/142*** (2016.01)    ***A23K 40/35*** (2016.01)
***A23K 50/10*** (2016.01)

(21) Application number: **21798059.8**

(22) Date of filing: **03.11.2021**

(52) Cooperative Patent Classification (CPC):
**A23K 40/35; A23K 20/142; A23K 50/10**

(86) International application number:
**PCT/EP2021/080471**

(87) International publication number:
**WO 2022/096487 (12.05.2022 Gazette 2022/19)**

(54) **COATED COMPOSITIONS OF BIOLOGICALLY ACTIVE INGREDIENTS FOR ORAL ADMINISTRATION TO RUMINANTS**

BESCHICHTETE ZUSAMMENSETZUNGEN BIOLOGISCH AKTIVER INHALTSSTOFFE ZUR ORALEN VERABREICHUNG AN WIEDERKÄUER

COMPOSITIONS ENROBÉES D'INGRÉDIENTS BIOLOGIQUEMENT ACTIFS POUR L'ADMINISTRATION ORALE À DES RUMINANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2020 EP 20206358**

(43) Date of publication of application:
**13.09.2023 Bulletin 2023/37**

(73) Proprietor: **Evonik Operations GmbH**
**45128 Essen (DE)**

(72) Inventors:
• **JACKSON, Barry**
**Pascagoula, Mississippi 39581 (US)**
• **PARYS, Claudia**
**35633 Lahnau (DE)**
• **RABE, Christian**
**63762 Großostheim (DE)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
**US-A- 5 080 917**

**Description**

Field of the Invention

The present invention relates to a composition for feeding a ruminant, the composition comprising a biologically active ingredient and a coating encapsulating the biologically active ingredient, a method for its preparation, a feed comprising the composition according to the present invention and a composition or the mixed feed according to the present invention for use in supplementing the diet of a ruminant with a biologically active ingredient.

Background of the Invention

Ruminant animals are mammals of the suborder *Ruminantia* including cattle, sheep, and goats. They have a stomach divided into four morphologically distinct compartments: the rumen, the reticulum, the omasum, and the abomasum. The rumen and the reticulum are derived from the terminal portion of the esophagus, and the omasum and abomasum are considered to be a genuine stomach. Bacteria present in the rumen enable ruminants to digest cellulosic materials such as grass to generate energy in form of volatile fatty acids, but they also attack and degrade partly other nutrients like starch, sugar and protein. Conventional digestion occurs in the abomasum, sometimes called the true stomach.

The rumen, which is essentially a continuous fermenter, contains a variety of microorganisms, which attack and degrade much of the feedstuffs consumed by a ruminant as part of their normal life cycle. Part of the ingested protein material is broken down in the rumen by enzymatic action of some microorganisms, others degrade carbohydrates like starch and sugar besides the above-mentioned bacteria which can break down even cellulose. A stream of ingested nutrients passes out of the rumen into the omasum, together with microbial cells. The function of the omasum is to separate liquids and solids. Much of the liquid reenters the rumen while the remainder of the material enters the abomasum. Digestion and absorption then proceed in the abomasum in a manner similar to that found in monogastrics. Enzymes secreted into the lumen of the abomasum digest much of the material, including the microbial cells. The digested microbial cells provide protein and amino acids to the ruminant.

The microbial action of the rumen has the great advantage of being able to convert many feed components, which have no direct nutritive value for the host into products, which can be assimilated and utilized by the host. For example, cellulose may be converted to a mixture of volatile fatty acids, which can serve as a source of energy to the host.

Unfortunately, this microbial action also presents certain disadvantages. For instance, proteins are broken down by the rumen microorganisms to peptides-and amino acids, and even further converted to carbon dioxide, volatile fatty acids, and ammonia. From these compounds, microbes can resynthesize microbial protein which typically has a higher nutritive value than feed protein. This process requires energy, provided by the degradation of carbohydrates.

All proteins present in animals are constituted by combinations of 20 different amino acids. Among these, ten essential amino acids are not adequately synthesized in the animal's body, and the animal must take them in. These ten amino acids are also referred to as essential amino acids. When essential amino acids are lacking in digestible protein of the ruminant diet, the ruminant's health, milk production, etc., are all negatively affected.

It is therefore common practice in ruminant production to supply biologically active ingredients in the daily diet of the animals in order to improve their conditions of health and their productive performance. Active ingredients of interest include amino acids, vitamins, enzymes, nutrients such as protein and carbohydrates, probiotic microorganisms, prebiotic foods, mineral salts, choline, guanidino acetic acid, etc. Some of these ingredients are already present in foods used for feeding animals. Sometimes the amount of essential active ingredients present in the diet may be insufficient or inadequate to cope with states of deficiency or situations of high productivity. Therefore, it is desirable to carefully formulate or supplement the daily diet of ruminant animals in order to address these concerns.

However, when physiologically active ingredients such as amino acids are orally fed, a substantial part of the ingredient, is decomposed by microorganisms in the rumen. This makes it difficult or even impossible for the animal to effectively utilize all or relevant amounts of the administered amino acids contained in the feed. Accordingly, supplemental amino acids are destroyed or converted and thus rendered unavailable for the growth of animals and animal production in general. However, animal production is limited by the supply of individual amino acids that should escape or bypass the rumen intact and reach the lower gastrointestinal tract of the animal, where they can be absorbed and become available for animal production. Accordingly, it is important to pass the biologically active ingredients through the rumen without decomposition by microorganisms to allow the biologically active ingredients to be effectively digested and absorbed in the abomasum and subsequent digestive tract.

Numerous methodologies were designed to increase the amount of a nutrient that passes through the rumen without being degraded by the rumen microflora, thereby delivering a larger portion of that nutrient to the lower gastrointestinal tract, including heat and chemical treatment, encapsulation and coating, use of amino acid analogs, and polymeric compounds of amino acids.

Examples for coated rumen protected products are granules or pellets of e.g. methionine, coated with polymers such as ethyl cellulose (EP 0 495 349) or vinylpyridine-styrene copolymers (US 4, 877, 621 and US 4,876,097).

The products described in EP 0 495 349 comprise a shaped particle of methionine and auxiliary substances which is coated with a protective shell of ethyl cellulose, preferably in a two-layer application with an inorganic auxiliary substance or filler (e.g. sodium aluminum silicate).

The products described in US 4,877,621 and in US 4,876,097, respectively, are based on coating of pellets which comprise amino acids and have diameters of 0.1 to approx. 5 mm, a pH-sensitive protective shell being built up. Since the intention is to produce pH-dependent release properties, a copolymer of 2-vinylpyridine and styrene is always used here as an essential constituent, and in US 4,876,097 additionally further components, such as e.g. zein, cellulose, aceto-butyrate, polyvinyl acetate, chitosan, ethyl cellulose and further auxiliary substances, including various solvents, which leads to a complicated protective shell comprising several components. The release properties indeed show entirely good results but are achieved at the expense of a high complexity.

US 5,080,917 discloses a coating agent for delaying the release of a physiologically active substance to be administered per os to ruminants, said coating comprising a veterinary acceptable watersoluble, synthetic high molecular compound and ethyl cellulose, said coating agent being stable in the first stomach or rumen of ruminants and capable of being effectively disintegrated for subsequent absorption in the $4^{th}$ stomach of ruminants, characterized in that said coating agent further comprises at least one substance which is miscible with both of said high molecular weight compound and ethyl cellulose and is insoluble in water.

EP 0 614 615 relates to a feedstuff's additive of a rumen-protected composition which comprises a biologically active substance and a protective shell and furthermore also has a pelleting-resistant protective shell of natural or synthetic polymers. Polymers which are suitable for this pelleting-resistant protective shell have an elasticity modulus at 30°C of between $10^8$ and $10^{11}$ dynes/cm$^2$ and a glass transition temperature of between 50°C and 150°C.

European Patent Application EP 1 360 904 A1 discloses coated active compound formulations having particle diameters of less than 1000 $\mu$m. Said formulation provides good rumen protecting rates and enables a stable and homogeneous distribution thereof in feed mixtures. On the other hand, granules or pellets with larger particle sizes (average particle size of approx. 1 - 2 mm) and a narrow particle size distribution enable a reduced coating amount and a more even distribution of the coating agent and hence better delivery and release properties.

The above polymer coatings have proven to be particularly effective for rumen protection. However, effective protection correlates with high consumption of expensive film-forming materials or coating compositions. In addition, the known products and corresponding methods of manufacture are often dissatisfying in terms of complexity of the chemical composition and/or the production method.

Coated compositions are also known in the field of pharmaceuticals or food supplements for humans.

However, the requirements for the coating material applied in pharmaceuticals or food supplements for humans are completely different from the requirements for the coating material applied to feed additives for ruminants: The digestive tract of humans and ruminants significantly differs especially with regard to residence times as well as biological and chemical environments (bacterial composition, pH). In humans, the residence time in the stomach is between 0.5 h and 3 h and in the small intestine between 7 h and 9 h. Accordingly, an appropriate half-life used to apply for sustained release methods in humans are typically three to four hours. In ruminants, part of the feed is still in the rumen after 6 h to 8 h. Accordingly, rumen protection rates (RPR) of $\geq$ 70 % after 6 h to 8 h are particularly desirable. Furthermore, the different pH levels in the human and the ruminant digestion tract and the application of active ingredients have to be considered. Typical human pharmaceuticals are protected just against the pH, which is acidic in the stomach, the release takes place in the small intestine at a pH above neutral. In case of a ruminant, the protection is against microbial degradation in the fore-stomachs, where the pH is close to neutral. The release can take place in the following parts of the gastro-intestinal tract which starts with an acidic environment in the abomasum.

Further, the ruminal activity has to be considered. Ruminal activity is the term used to describe a sequence of movements of the three fore-stomachs of ruminants. It serves to mix and transport the ingested feed and is mainly controlled by the parasympathetic nervous system. A cycle of ruminal activity begins with a double contraction of the reticulum. In the first contraction of the reticulum, its opening is still closed, the reticulum reduces its volume to half of its initial value. The second contraction leads to an almost complete contraction. The reticulo-omasal orifice is opened, and small feed components are transported to the omasum together with liquids. Immediately after the second contraction of the reticulum, the contraction wave of the front part of the upper rumen sac occurs. This transports the feed further into the next section of the digestive tract. Then, the dorsal (upper) and then the ventral (lower) rumen sac contracts twice. A comparable mechanical load is not present in humans. Accordingly, a rumen-protective coating must be stable enough to withstand the described ruminal activity.

In view of the above, it was a remaining need to provide new coatings for encapsulating biologically active compounds to be fed to a ruminant that can be easily applied onto an active compound core and that provides improved rumen protection and release properties/kinetics whilst requiring only a small amount of coating or film-forming material.

## Summary of the Invention

The inventors have unexpectedly found a coating composition comprising or consisting of a film-forming agent, which is ethyl cellulose, combined with an additive, selected from acetic ester of monoglycerides and triethyl citrate (TEC), that provides improved coating quality in terms of rumen protection and release properties vis-à-vis prior art coating compositions. Said coating can be easily applied onto an active compound core by common coating techniques. In addition, it was surprisingly found that by using the specific composition of film-forming agent and additive, the required amount of the expensive film-forming agent to be used can be significantly reduced.

Accordingly, one object of the present invention is a coated composition for feeding a ruminant, the composition for feeding a ruminant, the composition comprising:

A) a core comprising or consisting of a biologically active ingredient selected from the group consisting of i) amino acids, N-acylamino acids, N-guanylamino acids, and/or salts thereof and/or 2-hydroxy-4-(methyl)butyric acid calcium salt, ii) proteins, iii) peptides, iv) carbohydrates, v) vitamins, e.g., vitamin A, vitamin A acetate, vitamin A palmitate, vitamin B, thiamine, thiamine hydrochloride, riboflavin, nicotinic acid, nicotinic acid amide, calcium pantothenate, choline pantothenate, pyridoxine hydrochloride, choline chloride, cyano-cobalamin, biotin, folic acid, p-aminobenzoic acid, vitamin D2, vitamin D3, and vitamin E, vi) probiotic microorganisms, vii) prebiotic foods, viii) choline and salts thereof, ix) polyunsaturated fatty acids (PUFAs), and salts thereof, and x) any combination of the above components i) to ix); and

B) a coating encapsulating said core,

the coating constituting 1 to 15 % of the total weight of the coated composition,

and comprising or consisting of 85 to 95% wt./wt. of a film-forming agent and 5 to 15 % wt./wt. of an additive, wherein

the film-forming agent is ethyl cellulose; and

the additive is selected from an acetic ester of monoglycerides and triethyl citrate (TEC).

The composition according to the present invention enables particularly advantageous release kinetics for ruminants, i.e. RPRs $\geq$ 70 % after 6 h to 8 h. In view of such strongly delayed release and the associated stability of the composition in the presence of bacteria and in an acidic environment over a relatively long period of time, and also in view of the required mechanical stability (due to rumen motor activity), it would be expected that a particularly large amount of coating material would be required. Surprisingly, the opposite is the case and a coating amount of 1 to 15 % of the total weight of the coated composition suffices for effective protection, and at the same time prevents overprotection.

Further, the present invention provides a method for preparing the above coated composition, the method comprising the following steps:

a) providing core particles containing or consisting of a biologically active ingredient in a fluidized bed coater,
b) providing a coating mixture comprising a solvent, the film-forming agent and the additive,
c) heating the particles of step a) to a temperature in the range of 40°C to 90°C,
d) applying the mixture of step b) onto the heated particles of step c) via the spray nozzles of the fluidized bed coater to give a coated product,
e) drying the coated product obtained in step d), and
f) cooling the composition obtained in step e) or allowing the composition obtained in step e) to cool down.

Finally, the present invention provides a feed comprising the composition according to the present invention and a composition or the mixed feed according to the present invention for use in supplementing the diet of a ruminant with a biologically active ingredient.

## Detailed Description of the Invention

In context of the present invention, the term "biologically active ingredient" is used to denote any compound which has a functional or nutritive activity to a biological system, such as the organism of an animal, in particular of a ruminant, e.g. cattle, sheep or goat. Typically, a biologically active ingredient exhibits low stability and thus a reduced or even lost bio-effectiveness when exposed to unfavorable conditions, for example moisture, elevated temperature, oxygen and acidic and/or basic pH. When the biologically active ingredient is exposed to such conditions, it can for example decompose, dissociate, deactivate, and/or lose viability.

The term "biologically active ingredient" therefore refers to, for example, i) amino acids, such as lysine, methionine, tryptophan, arginine, histidine, isoleucine, leucine, phenylalanine, valine, and threonine, N-acylamino acids, e.g. N-acylmethionine, and N-guanylamino acids, e.g. N-guanylglycin also known as guinidinoacetic acid, and/or salts of thereof, such as lysine sulfate, lysine hydrochloride, and the salts of lysine with polyunsaturated fatty acids, and/or 2-hydroxy-4-(methylthio)butyric acid calcium salt, also known as the calcium salt of methionine hydroxy analogue (calcium MHA), ii) proteins, such as casein, corn proteins, and potato proteins, iii) peptides, such as oligo-polypeptides, e.g. methionyl-methionine (Met-Met) and/or polypeptides, iv) carbohydrates, such as starch, cane sugar, and glucose, v) vitamins, e.g., vitamin A, vitamin A acetate, vitamin A palmitate, vitamin B, thiamine, thiamine hydrochloride, riboflavin, nicotinic acid, nicotinic acid amide, calcium pantothenate, choline pantothenate, pyridoxine hydrochloride, choline chloride, cyano-cobalamin, biotin, folic acid, p-aminobenzoic acid, vitamin D2, vitamin D3, and vitamin E, vi) probiotic microorganisms, vii) prebiotic foods, viii) choline, and ix) polyunsaturated fatty acids (PUFAs), such as omega-3 fatty acids, e.g. alpha-linolenic acid (ALA), eicosapentanoic acid (EPA), and docosahexanoic acid (DHA) and salts thereof.

Preferably, the biologically active ingredient of the composition according to the present invention is an amino acid, such as lysine, methionine, tryptophan, arginine, histidine, isoleucine, leucine, phenylalanine, valine, and threonine, an N-acylamino acid, e.g. N-acylmethionine, and an N-guanylamino acid, e.g. N-guanylglycin also known as guinidinoacetic acid, and/or a salt thereof, such as lysine sulfate, lysine hydrochloride, and the salts of lysine with polyunsaturated fatty acids, and/or 2-hydroxy-4-(methylthio)butyric acid calcium salt, also known as the calcium salt of methionine hydroxy analogue (calcium MHA) or a peptide, such as an oligopolypeptide, e.g. methionyl-methionine (Met-Met) and/or polypeptides. In particular, the biologically active ingredient of the composition according to the present invention is lysine, methionine, guinidinoacetic acid, methionyl-methionine (Met-Met), and/or a salt thereof.

Preferably, the core of the composition according to the present invention consists of the biologically active ingredient itself. In this way, the loading of the composition according to the present invention with the biologically active ingredient may be further increased. However, there may be still cases where the core does not only consist of the biologically active ingredient itself. This is the case for example when further additives are necessary in order to provide granulates or spherical particles of the biologically active ingredient or when the biologically active ingredient in pure form is only poorly soluble in water and therefore has be converted into its acid addition salt such as lysine hydrochloride or lysine hydrosulfate.

Advantageously, the coating constitutes 2 to 8 % of the total weight of the coated composition and/or the coating comprises or consists of 86 to 93% wt./wt. of the film-forming agent and 7 to 14 % wt./wt. of the additive.

Deviations from any specific values, in particular values for weight percentages, explicitly mentioned herein, are also encompassed by the present invention, provided that they also lead to the effects achieved by the present invention.

In the context of the present invention, the terms "additive" and "plasticizer" are used interchangeably and have the same meanings. The terms "additive" and "plasticizer" refer to biocompatible compounds having the ability of increasing the plasticity of the film-forming agent. Plasticizers achieve through physical interaction with a polymer a reduction in the glass transition temperature and promote film formation, depending on the added amount. Suitable substances usually have a molecular weight of between 100 and 20 000 and comprise one or more hydrophilic groups in the molecule, e.g. hydroxyl, ester or amino groups.

The additive may comprise one or more of the additives indicated above.

However, in one specific embodiment according to the present invention, the coating comprises a single additive.

Preferably, the additive is triethyl citrate (TEC).

In one specific embodiment according to the present invention, the biologically active ingredient is selected from the group consisting of amino acids, N-acylamino acids, N-guanylamino acids,, salts of amino acids and/or 2-hydroxy-4-(methyl)butyric acid calcium salt,; the film-forming agent is ethyl cellulose (EC) and the additive is triethyl citrate (TEC).

As indicated above, the coating according to the present invention comprises or consist of a film-forming agent and an additive. The coating comprises or consist of 85 to 95% wt./wt., in particular 86 to 93% wt./wt. of the film-forming agent, and between 5 and 15 wt.-%, in particular between 7 and 14 wt.-% of the additive, each based on the total weight of the coating. The coating as a whole constitutes 1 to 15 %, in particular 2 to 8 % of the total weight of the coated composition.

In one specific embodiment according to the present invention, the film-forming agent is ethyl cellulose, and the additive is triethyl citrate. Preferably, ethyl cellulose is present in an amount of 85 to 95% wt./wt., in particular 86 to 93% wt./wt.; and triethyl citrate is present in an amount of between 5 and 15 wt.-%, in particular between 7 and 14 wt.-%, each based on the total weight of the coating. The coating as a whole constitutes 1 to 5%, in particular 2 to 8 % of the total weight of the coated composition.

The coating in the composition according to the present invention is not subject to any limitation regarding the number of layers. It may be desirable to apply more than one, e.g. two, three, or multiple layers of a coating as taught herein to prevent or conceal defects, e.g. cracks, and pores, formed in the coating during the preparation of the products. In addition, a mechanical impact on the products according to the present invention during their further handling may lead to micro-fissures or cracks in the outer layer. However, an overlap of the two or more layers significantly reduces or even avoids the danger of a potential leakage of the biologically active ingredient during the residence time of the products according to the present invention in the rumen. Thus, the presence of two or more layers in the coating of the products according to the present invention may also contribute to high yield of the rumen protected fraction of the biologically active ingredient. In one embodiment, the two or more layers of the coating each have a different composition, provided that the coating as such, comprising the two or more layers, comprises the amounts of film-forming agent and additive according to the present invention.

In one specific embodiment according to the present invention, the coating is a one-layer coating.

The core in the composition according to the present invention is not subject to any limitation regarding its shape. For example, the core may have a cylindrical or a spherical shape. Preferably, the core particles have a uniform shape and an even surface. Furthermore, a narrow particle size distribution of the core particles is advantageous.

Depending on the number of coating layers applied onto the core or particle comprising or consisting of the biologically active ingredient, the particle size of the compositions may be varied to obtain a given or desired particle size of the final product. It is preferred that the size of the compositions according to the present invention are such that they are not regurgitated or vomited by a ruminant upon ingestion.

Therefore, the average particle size of the compositions according to the present invention may be in the range of ca. 1 mm to ca. 6 mm, ca. 1.2 mm to ca. 5 mm, ca. 1.2 mm to ca. 4 mm, ca. 1.4 mm to ca. 3 mm, ca. 1.2 mm to ca. 2.8 mm, ca. 1.4 mm to ca. 2.6 mm, ca. 1.6 mm to ca. 2.4 mm, ca. 1.6 mm to ca. 2.2 mm or in the range of ca. 2 mm. Preferably, the compositions according to the present invention have an average particle size of at least 2 mm for reducing the chance of regurgitation or vomiting by a ruminant upon ingestion.

When preparing the product according to the present invention, it may be advantageous to add one or more additional ingredients to the coating as taught therein. In one specific embodiment according to the present invention, the coating consists only of the film-forming agent and an additive but does not comprise any additional ingredient. Preferably the film-forming agent according to this embodiment is ethyl cellulose and the additive according to this embodiment is triethyl citrate. Preferably, triethyl citrate is present in an amount of between 5 and 15 wt.-%, in particular between 7 and 14 wt.-% based on the total weight of the coating. The coating as a whole constitutes 1 to 5%, in particular 2 to 8 % of the total weight of the coated composition.

It may be advantageous to add further ingredient(s) to the core comprising the active ingredient, such as one or more ingredients selected from binding substances (e.g. cellulose derivatives such as hydroxy-propyl cellulose, methyl cellulose, sodium carboxymethylcellulose, vinyl derivatives such as polyvinyl alcohol or polyvinylpyrrolidone, gum arabicum, guaiac gum, sodium polyacrylate, and the like), filling substances (e.g. starch, proteins, crystalline cellulose and the like), inert ingredients (e.g. silica and silicate compounds), flow-control substances that help the formation of pellets, preservative agents (propionic acid or its salt, sorbic acid or its salt, benzoic acid or its salt, dehydroacetic acid or its salt, para-hydroxybenzoic acid esters, imazalil, thiabendazole, ortho-phenyl phenol, sodium ortho-phenyl phenol, diphenyl, and others compounds and mixtures thereof), antibacterial agent, and other compounds. The skilled person is familiar with techniques and compounds which are useful to achieve these purposes, and which are compatible with the production of the product according to the present invention.

It may also be advantageous to further enhance the nutritional value and/or the therapeutic value of the product according to the present invention by adding further feed ingredients (e.g. nutritional ingredients, veterinary or medicinal agents etc.) or other ingredients to the compositions as taught herein.

For instance, one or more ingredients selected from grain products, plant products, animal products, proteins (e.g. protein ingredients as obtained from sources such as dried blood or meat meal, meat and bone meal, cottonseed meal, soybean meal, rapeseed meal, sunflower seed meal, canola meal, safflower meal, dehydrated alfalfa, corn gluten meal, soybean protein concentrate, potato protein, dried and sterilized animal and poultry manure, fish meal, fish and poultry protein isolates, crab protein concentrate, hydrolyzed protein feather meal, poultry byproduct meal, liquid or powdered egg, milk whey, egg albumen, casein, fish solubles, cell cream, brewer's residues, and the like), mineral salts, vitamins (e.g. thiamine HCl, riboflavin, pyridoxine HCl, niacin, inositol, choline chloride, calcium pantothenate, biotin, folic acid, ascorbic acid, vitamin B12, p-aminobenzoic acid, vitamin A acetate, vitamin K, vitamin D, vitamin E, and the like), sugars and complex carbohydrates (e.g. water soluble and water-insoluble monosaccharides, disaccharides, and polysaccharides), veterinary compounds (e.g. promazine hydrochloride, chloromedoniate acetate, chloro-tetracycline, sulfamethazine, monensin, sodium monensin, poloxaline, oxytetracycline, BOVATEC, and the like), antioxidants (e.g. butylated hydroxyanisole, butylated hydroxytoluene, tertiary-butylhydroquinone, tocopherols, propyl gallate and ethoxyquin), trace element ingredients (e.g. compounds of cobalt, copper, manganese, iron, zinc, tin, nickel, chromium, molybdenum, iodine, chlorine, silicon, vanadium, selenium, calcium, magnesium, sodium and potassium and the like), and other compounds or ingredients, may be added to the product according to the present invention.

The skilled person is familiar with methods and ingredients that are suitable to enhance the nutritional and/or therapeutic or medicinal value of ruminant feeds, feed materials, premixes, feed additives, and feed supplements, and knows how to enhance the nutritional and/or therapeutic or medicinal value of the product according to the present invention.

In addition, or as an alternative, it is also possible to combine the product according to the present invention with a feed, feed material, or premix for feeding a ruminant. In context of the present invention the term premix or nutrient premix is used as known to the person skilled in the art and denotes a mixture comprising one or more ingredients such as vitamins, trace minerals, medicaments, feed supplements and diluents. The use of premixes has the advantage that a farmer who uses his own grain can formulate his own rations and be assured his animals are getting the recommended levels of minerals and vitamins.

Another object of the present invention is therefore a feed, feed material, or premix of feed additive for feeding a ruminant comprising the composition according to the present invention. Preferably, the premix further comprises a vitamin, trace mineral, feed supplements diluents, and/or medicaments, such as antibiotics, probiotics and/or prebiotics.

In principle, the application of the coating according to the present invention around a core comprising or consisting of a biologically active ingredient may be performed according to any suitable method known in the art, such as drum coating or fluidized bed coating. However, it was found that the best method of providing a biologically active ingredient with the coating according to the present inventions is fluidized bed coating.

A further object of the present invention in the provision of a method for preparing a coated composition as described above, the method comprising the following steps:

a) providing core particles containing or consisting of a biologically active ingredient in a fluidized bed coater,
b) providing a coating mixture comprising a solvent, the film-forming agent and the additive,
c) heating the particles of step a) to a temperature in the range of 40°C to 90°C, in particular to 45°C to-80°C, preferably to 45°C to 70°C;
d) applying the mixture of step b) onto the heated particles of step c) via the spray nozzles of the fluidized bed coater to give a coated product,
e) drying the coated product obtained in step d), and
f) cooling the composition obtained in step e) or allowing the composition obtained in step e) to cool down.

In case the coated composition to be prepared is to have two or more layers, steps c) to d) may be repeated with the composition obtained in step e) accordingly.

The term "core particles" is to be understood as defined above.

In order to obtain the coating solution of step b), the film-forming agent, the additive and optionally further auxiliary ingredients are admixed with in a suitable solvent. Both, aqueous and organic solvents may be used.

In one embodiment, the solvent is an organic solvent. Said organic solvent may be selected from saturated, non-saturated and aromatic hydrocarbon compounds, short-chain alcohols, ketones or mixtures of the aforementioned solvents. Suitable alcohols are e.g. ethanol and iso-propanol, or mixtures thereof. An example for a suitable ketone compound is acetone. Preferably, the solvent to be used is a short-chain alcohol, in particular ethanol.

In one specific embodiment according to the present invention, the film-forming agent and the additive were dissolved in ethanol. Preferably the film-forming agent according to this embodiment is ethyl cellulose and the additive according to this embodiment is triethyl citrate.

It was further found that administering to a ruminant the composition according to the present invention, a composition obtained or obtainable by a process according to the present invention and/or a feed, feed material, premix or feed additive according to the present invention is suitable to compensate amongst others for a lack of nutrients and essential amino acids in a ruminant diet.

Yet a further object of the present invention is therefore a composition according to the present invention and/or a feed, feed material, premix or feed additive according to the present invention for use in supplementing the diet of a ruminant with a biologically active ingredient, wherein said composition and/or feed, feed material, premix or feed additive is provided to the ruminant.

In the following, the present invention will be more particularly elucidated below on the basis of exemplary and non-limiting embodiments.

## Examples

### I. Material and methods

The below coating experiments were carried out in conventional fluidized bed coating units for laboratory scale (test series 1, 2 and 4) and for production scale (test series 3), respectively.

**Chemicals and reagents:**

<u>Active ingredients</u>

Biolys®; Methionine (Evonik Industries)

<u>Film-forming agent</u>

Ethyl cellulose (PHT International Inc.)

<u>Additives</u>

Acetic ester of monoglycerides (commercial product Acetem 95 Co, Danisco)

Triethyl citrate (Sigma Aldrich)

**Analytical methods:**

For assessing the coating quality, the rumen protection rate (RPR) was determined for the test samples.

<u>*In vitro* test</u>

The RPR corresponds to the content of still protected biologically active ingredient after an incubation time of 6 h to 24 h in a test medium (water pH 5.5 or buffer solution). The 6 h value is a qualitative measure for the protection rate. The 24 h value does not reflect the real processes in the animal but is an indication whether the tested sample may be over-protected.

The coated composition is shaken in desalinated water at 37°C / pH 5.5 in a shaking water bath.

Desired value for RPR:

| | |
|---|---|
| for 6 h (rumen) | high ($\geq$ 70 %) |
| for 24 h (rumen and further digestive tract) | low (approx. $\leq$ 50%) |

After the particular period of time in the vibrating water bath, the solutions were filtered, the biologically active ingredient released from the coated composition was determined quantitatively in the filtrate and the protection rate was calculated according to the following formula:

$$RPR [\%] = 1- (\text{amount of released active ingredient / starting amount of active ingredient}) *100$$

<u>*In situ* method for testing digestibility of coated active ingredients in the rumen</u>

*Nylon bags*: ANKOM, type R510 (5x10 cm) for concentrates.

*Preparation*: After labeling, the bags were washed before used for the first time by using a washing machine choosing the cold wash program within a laundry net (the same program that will be used later for the incubated bags). After washing, the bags were dried at 60 °C for 12 hours in a forced air oven, then cooled down in a desiccator and subsequently weighted to get their empty weight.

*Sealing the bags*: Heat-sealing

**Preparation of feeds and initial weight**

Dry Matter (DM) content was measured first (103 °C for 4 hours).

The initial weight was 1.5 g for active ingredient per bag. The ratio of initial weight to bag surface should be 12,5 mg DM/cm$^2$ on average.

**Incubation of the bags in the rumen**

The incubation period was 8 h (4 bags per animal). The determination of wash-out losses in the washing machine is done with 3 bags per feed.

All bags were incubated simultaneously at the same time and removed at the same time (subsequent incubation sequences). The bags were equally distributed at the cylinder or item that is put into the rumen.

Incubation started directly before morning feeding. Before incubation, bags were put for 1 min in warm water (40 °C) for conditioning.

**Removing and treatment of the bags after the incubation**

After removing the bags from the rumen, they were put into iced water, afterwards roughly rinsed with cold tap water and then washed in the washing machine (in a laundry net). The rinsed bags can also be frozen or stored for a maximum of 24 hours before they are washed. After the washing machine, the bags were dried or stored frozen until drying.

In the final step, the incubated bags were dried at 40°C for 24 hours in a drying oven with circulating air (and cooled down in a desiccator for weighting).

## II. Coating Experiments

Test series 1:

An ethanolic solution of the film-former ethyl cellulose (EC) was prepared with a solids content of 7.1 wt.%. To this solution, either triethyl citrate (TEC, Example 1) or the acetic ester of monoglycerides (commercial product Acetem 95 Co, Danisco; Example 2) was added under stirring in order to obtain the coating composition as a sprayable solution. The amount of additive was 10% of the dry ethyl cellulose mass. Thereby, a coating comprising 9 wt.% TEC additive and 91 wt.% of EC (film forming agent) was obtained.

A coating solution without additive was prepared for comparison purposes.

Subsequently, 3 kg Biolys ® granules (sieve fraction 1.6 - 2 mm) were introduced into the fluidized bed coater and heated to a starting temperature of T = 50 °C. After having reached the starting temperature, the coating process was started, and the respective coating solutions were sprayed on the granules. In total, a coating amount of 10 wt.-% (based on the total weight of the coated composition) was applied. Accordingly, a coating comprising 9 wt.% TEC additive and 91 wt.% of EC (film forming agent) was obtained. After having reached the desired coating amount, the spraying was finished, the coated product was dried and then removed from the coater.

The RPR was determined for the thus-obtained samples via the above-described *in vitro* test. The results are summarized in Table 1 and show the lysine retention after 6h and 24 h, respectively.

**Table 1**

| Sample | RPR after 6h [%] | RPR after 24h [%] |
|---|---|---|
| Comparative Example 1 (without additive) | 67 | 35 |
| Example 1 (additive: TEC) | 76 | 39 |
| Example 2 (additive: Acetem 95 Co) | 71 | 36 |

As derivable from the results presented in Table 1, the addition of an additive in the above-specified amounts enables a recognizable improvement of the rumen protection rate, especially for the 6h value where PRPs > 70% could be achieved. A slightly higher protection rate is achieved with triethyl citrate as additive. The 24 h value shows that, although higher protection rates were achieved, no over-protection of the granules occurred.

Test series 2:

In test series 2, the examples were carried out in an analogous manner as in test series 1, except that the amount of the ethyl cellulose -based coating was varied in the range of 5 - 10 wt.-% (based on the total weight of the coated composition). The additive portion in the coating was adjusted again to 10% (in relation to dry ethyl cellulose). Thereby, a coating comprising 9 wt.% TEC additive and 91 wt.% of EC (film forming agent) was obtained. Analyses were carried out in the same way as in test series 1. The results were compiled in table 2.

**Table 2**

| Sample | Amount of ethyl cellulose-based coating [%] | 5 | 7,5 | 10 |
|---|---|---|---|---|
| Comparative Example 1 (without additive) | RPR after 6 h [%] | 43 | 55 | 65 |
| | RPR after 24 h [%] | 18 | 24 | 30 |
| Example 1 (additive: TEC) | RPR after 6 h [%] | 49 | 64 | 76 |
| | RPR after 24 h [%] | 24 | 32 | 39 |
| Example 2 (additive: Acetem 95 Co) | RPR after 6 h [%] | 47 | 61 | 71 |
| | RPR after 24 h [%] | 22 | 31 | 36 |

Based on the results shown in Table 2, it is evident that with the addition of an additive in the above-specified amounts, the protection of the active ingredient can be improved, even at lower coating amounts. A slightly higher protection rate is achieved with triethyl citrate as additive.

In case the ethyl cellulose-based coating constitutes more than 15 % of the total weight of the coated composition, too little of the protected active ingredient will be released and available to the animal ("overprotection").

Comparing the RPR results for 6 h for the sample without additive addition versus those with admixture of triethyl citrate, a saving of coating agent can be estimated. With addition of triethyl citrate approx. 2 % less coating agent are required, to obtain comparable protection rates. This results to a reduction of a specific amount in the range of 20 - 30 g ethyl cellulose/kg pellet. Accordingly, the amount of "non-natural" ingredients contained in the animal feed is significantly reduced.

Test series 3:

An ethanolic solution of the film-former ethyl cellulose (EC) was prepared with a solids content of 7.1 wt.%. To this solution, triethyl citrate (TEC) was added under stirring in order to obtain the coating composition as a sprayable solution. The amount of additive was 10% of the dry ethyl cellulose mass. Thereby, a coating comprising 9 wt.% TEC additive and 91 wt.% of EC (film forming agent) was obtained.

A coating solution without additive was prepared for comparison purposes.

Subsequently, 350 kg of uncoated pelleted methionine (cylindrical pellets having a diameter of 1.6 to 1.8 mm and a length of 2 to 3 mm) was introduced into the fluidized bed coater and heated to a starting temperature of T = 55 °C. Methods for pelleting of methionine may be found e.g. in EP 0 495 349. After having reached the starting temperature, the coating process was started and the respective coating solutions were sprayed on the granules. In total, a coating amount of 4.2% the above-described ethyl cellulose-based coating (based on the total weight of the coated composition) was applied. After having reached the desired coating amount, the spraying was finished, the coated product was dried and then removed from the coater.

The RPR was determined for the thus-obtained samples via the above-described *in vitro* and *in situ* tests. The results are compiled in table 3.

**Table 3**

| Sample | *in vitro* | | *in situ* |
|---|---|---|---|
| | RPR after 6 h /[%] | RPR after 24 h [%] | RPR after 8 h [%] |
| **Comparative Example (without additive)** | 80 | 40 | 75 |
| **Example 1 (additive: TEC)** | 83 | 48 | 79 |

In accordance with test series 1 and 2, the results for coated methionine indicate that by adding triethyl citrate (TEC), the rumen protection rate can be improved vis-à-vis the reference product (RPR after 6 h and 8 h, respectively). Both the laboratory results and the results of the *in situ* tests show a higher methionine active substance protection. At the same time, good release rates after 24 h are ensured (meaning that "overprotection" is avoided).

The skilled person will understand that due to its high hygroscopy / sensitivity towards water, lysine requires slightly higher amounts of the ethyl cellulose-based coating in order to achieve desirable RPR values.

Test series 4:

For comparison reasons the test series 1 was extended with investigation of the impact of an increased additive addition, esp. for triethyl citrate. Starting from a concentration of 10 wt.% TEC (based on the amount of dry ethyl cellulose, corresponding to a coating composition comprising 9 wt.% TEC and 91 wt.% EC), in the test series 4 the TEC amount was increased to 15 wt. % (corresponding to a coating composition comprising 13 wt.% TEC and 87 wt.% EC) and 20 wt.% (corresponding to a coating composition comprising 16,7 wt.% TEC and 83,3 wt.% EC) respectively.

During processing it was observed that with higher TEC concentration, the particles turned more sticky, meaning that more agglomerates were generated.

The resulting RPR values from the in vitro analyses are summarized in table 4.

**Table 4**

| Sample | *in vitro* | |
| --- | --- | --- |
| | RPR after 6 h /[%] | RPR after 24 h [%] |
| **Example 1** 9 wt.% TEC; 91 wt.% EC | 76 | 39 |
| **Example 2** 13.5 wt.% TEC; 86.5 wt.% EC | 71 | 33 |
| **Example 3** 18 wt.% TEC; 82 wt.% EC | 66 | 27 |

As derivable from the results presented in Table 4, an almost linear decrease of the protection rate with increase of TEC concentration occurs. It is thus evident that an amount of additive ≤14 wt.% is particularly desirable.

The 24 h release rates are also efficient in each of Examples 1 to 3.

**Claims**

1. A coated composition for feeding a ruminant, the composition comprising:

   A) a core comprising or consisting of a biologically active ingredient selected from the group consisting of i) amino acids, N-acylamino acids, N-guanylamino acids, and/or salts thereof and/or 2-hydroxy-4-(methyl)butyric acid calcium salt, ii) proteins, iii) peptides, iv) carbohydrates, v) vitamins, vi) probiotic microorganisms, vii) prebiotic foods, viii) choline and salts thereof, ix) polyunsaturated fatty acids (PUFAs), and salts thereof, and x) any combination of the above components i) to ix); and
   B) a coating encapsulating said core,

   the coating constituting 1 to 15 % of the total weight of the coated composition,
   and comprising or consisting of 85 to 95% wt./wt. of a film-forming agent and 5 to 15 % wt./wt. of an additive, wherein
   the film-forming agent is ethyl cellulose; and
   the additive is selected from an acetic ester of monoglycerides and triethyl citrate (TEC).

2. The coated composition according to claim 1, wherein the coating constitutes 2 to 8 % of the total weight of the coated composition.

3. The coated composition according to claim 1 or 2, wherein the coating comprises or consist of 86 to 93% wt./wt. of the film-forming agent and 7 to 14 % wt./wt. of the additive.

4. The coated composition according to any of claims 1 to 3, wherein the film-forming agent is ethyl cellulose, and the additive is triethyl citrate.

5. The coated composition according to any of claims 1 to 4, wherein the coating is a one-layer coating.

6. The coated composition according to any of claims 1 to 4, wherein the composition has two or more layers of the coating, wherein each has a different composition, provided that the coating as such, comprising the two or more layers, comprises the amounts of the film-forming agent and additive according to any of claims 1 to 3.

7. The coated composition according to any of claims 1 to 6, wherein the particle size of the coated composition is in the range of 1 mm to 6 mm.

8. A method for preparing a coated composition according to any of claims 1 to 7, the method comprising the following steps:

a) providing core particles containing or consisting of a biologically active ingredient in a fluidized bed coater,
b) providing a coating mixture comprising a solvent, the film-forming agent and the additive,
c) heating the particles of step a) to a temperature in the range of 40°C to 90°C,
d) applying the mixture of step b) onto the heated particles of step c) via the spray nozzles of the fluidized bed coater to give a coated product,
e) drying the coated product obtained in step d), and
f) cooling the composition obtained in step e) or allowing the composition obtained in step e) to cool down.

9. The method according to claim 8, wherein the steps c) to d) are repeated with the composition obtained in step e), in case the coated composition to be prepared is to have two or more layers.

10. The method according to claim 8 or 9, wherein the temperature in step c) is in the range of 45°C to 80°C, preferably 45°C to 70°C.

11. The method according to any of claims 8 to 10, wherein aqueous or organic solvents are used.

12. The method according to any of claims 8 to 11, wherein the solvent is an organic solvent selected from saturated, non-saturated and aromatic hydrocarbon compounds, short-chain alcohols, ketones or mixtures of the aforementioned solvents.

13. The method according to claim 11, wherein the alcohol is ethanol, iso-propanol or a mixture thereof.

14. Feed, feed material, or premix of feed additive for feeding a ruminant comprising a composition according to any one of claims 1 to 7.

15. A composition according to any of claims 1 to 7 and/or a feed, feed material, or premix of feed additive according to claim 14 for use in supplementing a diet of a ruminant with a biologically active ingredient, wherein said composition and/or feed, feed material, or premix of feed additive is provided to the ruminant.


**Patentansprüche**

1. Beschichtete Zusammensetzung zum Füttern eines Wiederkäuers, wobei die Zusammensetzung Folgendes umfasst:

A) einen Kern, der einen aus der aus i) Aminosäuren, N-Acylaminosäuren, N-Guanylaminosäuren und/oder Salzen davon und/oder 2-Hydroxy-4-(methyl)buttersäure-Calciumsalz, ii) Proteinen, iii) Peptiden, iv) Kohlenhydraten, v) Vitaminen, vi) probiotischen Mikroorganismen, vii) präbiotischen Nahrungsmitteln, viii) Cholin und Salzen davon, ix) mehrfach ungesättigten Fettsäuren (Polyunsaturated Fatty Acids, PUFAs) und Salzen davon und x) einer beliebigen Kombination der obigen Komponenten i) bis ix) bestehenden Gruppe ausgewählten biologisch aktiven Bestandteil umfasst oder daraus besteht; und
B) eine den Kern umhüllende Beschichtung,

wobei die Beschichtung 1 bis 15 % des Gesamtgewichts der beschichteten Zusammensetzung ausmacht und 85 bis 95 Gew.-% eines Filmbildners und 5 bis 15 Gew.-% eines Additivs umfasst oder daraus besteht, wobei es sich bei dem Filmbildner um Ethylcellulose handelt; und
das Additiv aus einem Essigsäureester von Monoglyceriden und Triethylcitrat (TEC) ausgewählt ist.

2. Beschichtete Zusammensetzung nach Anspruch 1, wobei die Beschichtung 2 bis 8 % des Gesamtgewichts der beschichteten Zusammensetzung ausmacht.

**3.** Beschichtete Zusammensetzung nach Anspruch 1 oder 2, wobei die Beschichtung 86 bis 93 Gew.-% an Filmbildners und 7 bis 14 Gew.-% an Additiv umfasst oder daraus besteht.

**4.** Beschichtete Zusammensetzung nach einem der Ansprüche 1 bis 3, wobei es sich bei dem Filmbildner um Ethylcellulose handelt und es sich bei dem Additiv um Triethylcitrat handelt.

**5.** Beschichtete Zusammensetzung nach einem der Ansprüche 1 bis 4, wobei es sich bei der Beschichtung um eine einschichtige Beschichtung handelt.

**6.** Beschichtete Zusammensetzung nach einem der Ansprüche 1 bis 4, wobei die Zusammensetzung zwei oder mehr Schichten der Beschichtung aufweist, wobei jede eine unterschiedliche Zusammensetzung hat, mit der Maßgabe, dass die Beschichtung als solche, die die zwei oder mehr Schichten umfasst, die Mengen an Filmbildner und Zusatzstoff nach einem der Ansprüche 1 bis 3 umfasst.

**7.** Beschichtete Zusammensetzung nach einem der Ansprüche 1 bis 6, wobei die Teilchengröße der beschichteten Zusammensetzung im Bereich von 1 mm bis 6 mm liegt.

**8.** Verfahren zur Herstellung einer beschichteten Zusammensetzung nach einem der Ansprüche 1 bis 7, wobei das Verfahren die folgenden Schritte umfasst:

a) die Bereitstellung von einen biologisch aktiven Bestandteil enthaltenden oder daraus bestehenden Kernpartikeln in einem Wirbelschichtbeschichter,
b) die Bereitstellung einer ein Lösungsmittel, den Filmbildner und das Additiv umfassenden Beschichtungsmischung,
c) das Erhitzen der Partikel aus Schritt a) auf eine Temperatur im Bereich von 40 °C bis 90 °C,
d) das Aufbringen der Mischung aus Schritt b) auf die erhitzten Partikel aus Schritt c) über die Sprühdüsen des Wirbelschichtbeschichters unter Erhalt eines beschichteten Produkts,
e) das Trocknen des in Schritt d) erhaltenen beschichteten Produkts und
f) das Abkühlen der in Schritt e) erhaltenen Zusammensetzung oder das Abkühlenlassen der in Schritt e) erhaltenen Zusammensetzung.

**9.** Verfahren nach Anspruch 8, wobei die Schritte c) bis d) mit der in Schritt e) erhaltenen Zusammensetzung wiederholt werden, wenn die herzustellende beschichtete Zusammensetzung zwei oder mehr Schichten aufweisen soll.

**10.** Verfahren nach Anspruch 8 oder 9, wobei die Temperatur in Schritt c) im Bereich von 45 °C bis 80 °C, vorzugsweise 45 °C bis 70 °C, liegt.

**11.** Verfahren nach einem der Ansprüche 8 bis 10, wobei wässrige oder organische Lösungsmittel verwendet werden.

**12.** Verfahren nach einem der Ansprüche 8 bis 11, wobei es sich bei dem Lösungsmittel um ein aus gesättigten, ungesättigten und aromatischen Kohlenwasserstoffverbindungen, kurzkettigen Alkoholen, Ketonen oder Mischungen der oben erwähnten Lösungsmittel ausgewähltes organisches Lösungsmittel handelt.

**13.** Verfahren nach Anspruch 11, wobei es sich bei dem Alkohol um Ethanol, Isopropanol oder eine Mischung davon handelt.

**14.** Futter, Futtermaterial oder Vormischung von Futteradditiv zum Füttern eines Wiederkäuers, umfassend eine Zusammensetzung nach einem der Ansprüche 1 bis 7.

**15.** Zusammensetzung nach einem der Ansprüche 1 bis 7 und/oder Futter, Futtermaterial oder Vormischung von Futteradditiv nach Anspruch 14 zur Verwendung bei der Ergänzung der Ernährung eines Wiederkäuers mit einem biologisch aktiven Bestandteil, wobei man dem Wiederkäuer die Zusammensetzung und/oder das Futter, das Futtermaterial oder die Vormischung des Futteradditivs bereitstellt.

**Revendications**

**1.** Composition revêtue pour l'alimentation d'un ruminant, la composition comprenant :

A) un noyau comprenant ou constitué d'un ingrédient biologiquement actif sélectionné dans le groupe constitué par i) des acides aminés, des N-acylamino-acides, des N-guanylamino-acides, et/ou des sels correspondants et/ou un sel de calcium d'acide 2-hydroxy-4-(méthyl)butyrique, ii) des protéines, iii) des peptides, iv) des glucides, v) des vitamines vi) des micro-organismes probiotiques, vii) des aliments prébiotiques, viii) de la choline et des sels correspondants, ix) des acides gras polyinsaturés (PUFA) et des sels correspondants, et x) une quelconque combinaison des composants ci-dessus i) à ix) ; et

B) un revêtement encapsulant ledit noyau,

le revêtement constituant 1 à 15 % du poids total de la composition revêtue,

et comprenant ou constitué de 85 à 95 % en poids/poids d'un agent de formation de film et 5 à 15 % en poids/poids d'un additif, dans laquelle

l'agent de formation de film est une éthylcellulose ; et

l'additif est sélectionné parmi un ester acétique de monoglycérides et le citrate de triéthyle (TEC).

2. Composition revêtue selon la revendication 1, dans laquelle le revêtement constitue 2 à 8 % du poids total de la composition revêtue.

3. Composition revêtue selon la revendication 1 ou 2, dans laquelle le revêtement comprend ou est constitué de 86 à 93 % en poids/poids de l'agent de formation de film et 7 à 14 % en poids/poids de l'additif.

4. Composition revêtue selon l'une quelconque des revendications 1 à 3, dans laquelle l'agent de formation de film est une éthylcellulose et l'additif est le citrate de triéthyle.

5. Composition revêtue selon l'une quelconque des revendications 1 à 4, dans laquelle le revêtement est un revêtement monocouche.

6. Composition revêtue selon l'une quelconque des revendications 1 à 4, dans laquelle la composition possède deux couches ou plus du revêtement, dans laquelle chacune a une composition différente, à condition que le revêtement en tant que tel, comprenant les deux couches ou plus, comprend les quantités d'agent de formation de film et d'additif selon l'une quelconque des revendications 1 à 3.

7. Composition revêtue selon l'une quelconque des revendications 1 à 6, dans laquelle la taille de particule de la composition revêtue est dans la plage de 1 mm à 6 mm.

8. Procédé de préparation d'une composition revêtue selon l'une quelconque des revendications 1 à 7, le procédé comprenant les étapes suivantes :

a) fourniture de particules de noyau contenant ou constituées d'un ingrédient biologiquement actif dans un dispositif de revêtement à lit fluidisé,

b) fourniture d'un mélange de revêtement comprenant un solvant, l'agent de formation de film et l'additif,

c) chauffage des particules de l'étape a) jusqu'à une température dans la plage de 40 °C à 90 °C,

d) application du mélange de l'étape b) sur les particules chauffées de l'étape c) via les buses de pulvérisation du dispositif de revêtement à lit fluidisé pour donner un produit revêtu,

e) séchage du produit revêtu obtenu à l'étape d), et

f) refroidissement de la composition obtenue à l'étape e) ou le fait de laisser refroidir la composition obtenue à l'étape e) .

9. Procédé selon la revendication 8, dans laquelle les étapes c) à d) sont répétées avec la composition obtenue à l'étape e), dans le cas où la composition revêtue à préparer doit avoir deux couches ou plus.

10. Procédé selon la revendication 8 ou 9, dans lequel la température à l'étape c) est dans la plage de 45 °C à 80 °C, de préférence 45 °C à 70 °C.

11. Procédé selon l'une quelconque des revendications 8 à 10, dans lequel des solvants aqueux ou organiques sont utilisés.

12. Procédé selon l'une quelconque des revendications 8 à 11, dans lequel le solvant est un solvant organique sélectionné parmi des composés hydrocarbonés saturés, non saturés et aromatiques, des alcools à chaîne courte, des cétones ou des mélanges des solvants mentionnés ci-dessus.

13. Procédé selon la revendication 11, dans lequel l'alcool est l'éthanol, l'isopropanol ou un mélange correspondant.

14. Produit alimentaire pour animaux, matériau de produit alimentaire pour animaux ou prémélange d'additif de produit alimentaire pour animaux pour l'alimentation d'un ruminant comprenant une composition selon l'une des revendications 1 à 7.

15. Composition selon l'une quelconque des revendications 1 à 7 et/ou produit alimentaire pour animaux, matériau de produit alimentaire pour animaux ou prémélange d'additif de produit alimentaire pour animaux selon la revendication 14 pour une utilisation en complément d'un régime d'un ruminant avec un ingrédient biologiquement actif, dans laquelle ladite composition et/ou produit alimentaire pour animaux, matériau de produit alimentaire pour animaux ou prémélange d'additif de produit alimentaire pour animaux est fourni au ruminant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0495349 A
- US 4877621 A
- US 4876097 A
- US 5080917 A
- EP 0614615 A
- EP 1360904 A1